Europäisches Patentamt

European Patent Office

Office européen des brevets

(11) Veröffentlichungsnummer: **0 176 077**
**B1**

(12) **EUROPÄISCHE PATENTSCHRIFT**

(45) Veröffentlichungstag der Patentschrift:
16.03.88

(51) Int. Cl.⁴: **C 07 D 323/00**

(21) Anmeldenummer: **85112085.7**

(22) Anmeldetag: **24.09.85**

(54) **Verfahren zur Isolierung von makrozyklischen Polyethern.**

(30) Priorität: **24.09.84 DE 3435017**

(43) Veröffentlichungstag der Anmeldung:
**02.04.86 Patentblatt 86/14**

(45) Bekanntmachung des Hinweises auf die Patenterteilung:
**16.03.88 Patentblatt 88/11**

(84) Benannte Vertragsstaaten:
**BE DE FR GB IT NL**

(56) Entgegenhaltungen:
**US-A-4 436 923**

(73) Patentinhaber: **Consortium für elektrochemische Industrie GmbH, Zielstattstrasse 20, D-8000 München 70 (DE)**

(72) Erfinder: **Gamon, Norbert, Dr. Dipl.- Chem., Mühlbachstrasse 52, D-8261 Emmerting (DE)**

## Beschreibung

Die Erfindung betrifft ein Verfahren zum Isolieren makrozyklischer Polyether aus deren Gemischen mit offenkettigen Polyglycolen durch Extraktion mit gesättigem Kohlenwasserstoff.

Bei der Synthese von makrozyklischen Polyethern, im folgenden auch als Kronenether bezeichnet, finden sich im Reaktionsgemisch zumeist offenkettige Polyglycole. Derartige Polyglycole liegen im Reaktionsgemisch z. B. als nicht vollständig umgesetzte Ausgangsverbindungen oder als Folgeprodukte von Glycoleinheiten aufweisenden Halogeniden, Tosylaten und dergleichen vor. Die Trennung dieser Polyglycole von den an sich erwünschten Kronenethern bereitet erhebliche Schwierigkeiten, da ihr Siedewerhalten und auch ihre Löslichkeit in bekannten Extraktionsmitteln denen der Kronenether sehr ähnlich ist.

In SYNTH. COMM. 9, 851 (1979) wurde bereits die Entfernung von hydroxylgruppenhaltigen Verbindungen aus deren Gemischen mit 18-Krone-6 durch Extraktion mit kaltem Pentan beschrieben. Unter diesen Bedingungen werden jedoch Kronenether nur sehr langsam und mit unbefriedigender Reinheit extrahiert.

Es wurde nun gefunden, daß Kronenether durch Extraktion mit gesättigten Kohlenwasserstoffen in hoher Reinheit gewonnen werden, wenn dem zu extrahierenden Reaktionsgemisch mindestens 10 Gewichtsprozent Ethylenglycol zugegeben werden.

Gegenstand der Erfindung ist ein Verfahren zur Isolierung von makrozyklischen Polyethern aus deren Gemischen mit offenkettigen Polyglycolen durch Extraktion mit gesättigten Kohlenwasserstoffen, das dadurch gekennzeichnet ist daß die Extraktion in Gegenwart von mindestens 10 Gewichtsprozent Ethylenglycol, bezogen auf das Gewicht des Gemisches an makrozyklischem Polyether/offenkettiger Polyglycolen, durchgeführt wird.

Vorzugsweise beträgt die Menge an eingesetztem Ethylenglycol bezogen auf die Menge des Gemisches makrozyklischer Polyether/ offentkettigerPolyglycole 50 bis 200 Gewichtsprozent.

Die erfindungsgemäß aus dem Reaktionsgemisch zu isolierenden makrozyklischen Polyether sind insbesondere solche die aus 5 bis 10 Ehtylenoxid-Einheiten aufgebaut sind. Diese Ethylenoxid-Einheiten weisen gegebenenfalls Substituenten auf. Beispiele für derartige Substituenten sind Methyl Ethyl, n-Propyl-, n-Butyl-, Allyl-, Crotyl-, Prenyl-, Phenyl-, Benzyl-, Methoxymethyl-, Ethoxymethyl-, u.a.

Individuelle Beispiele für erfindungsgemäß zu isolierende Kronenether sind 18-Krone-6, Methyl-18-Krone-6, Ethyl-18-Krone-6, Propyl-18-Krone-6, 15-Krone-5, Allyloxymethyl-18-Krone-6, 1-Aza-18-Krone-6, N-Methyl-1- aza-18-Krone-6, N-Allyl -1- aza-18-Krone-6, insbesondere 18-Krone-6.

Die durch Extraktion abzutrennenden offenkettigen Polyglycole sind insbesondere solche die die gleichen Struktureinheiten aufweisen, wie die zu isolierenden Kronenether. Beispiele für derartige Glycole sind Triethylenglycol, Tetraethylenglycol, Pentaethylenglycol, Hexaethylenglycol, Heptaethylenglycol, Octaethylenglycol, Decaethylenglycol, 1-Methyl-Tetraethylenglycol, 1-Allyl-Tetraethylenglycol, 1-Phenyl-Tetraethylenglycol, Diethanolamin, N-Methyldiethanolamin, N-Allyl-diethanolamin.

Beispiele für erfindungsgemäß zur Extraktion einzusetzende gesättigte Kohlenwasserstoffe sind geradkettige und verzweigte Alkane, Zykloalkane sowie deren Gemische, insbesondere n-Pentan, n-Hexan, n-Heptan, n-Oktan, n-Nonan, n-Dekan, i-Pentan, i-Oktan, Cyclopentan, Cyclohexan, Methylcyclohexan u.a.

Die Extraktionstemperatur betragen in der Regel 20°C bis 150°C, insbesondere 40°C bis 100°C.

Die Extraktionen werden nach Methoden durchgeführt, die auch bereits bisher zur Flüssigextraktion angewandt wurden. Es kann diskontinuierlich oder kontinuierlich extrahiert werden. In einer vorteilhaften Ausführungsform des Verfahrens wird kontinuierlich im Gegenstrom extrahiert. Beispielsweise wird eine Pulsations-Füllkörperkolonnverwendet, wobei das Extraktionsmittel, z.B Cyclohexan, am Kolonnenfuß, Ethylenglycol am Kolonnenkopf und die Kronenetherhaltige Mischung im oberen Drittel der Kolonne zugeführt wird.

Gegebenenfalls nach Trennung der Phasen und nach Abziehen des Extraktionsmittels wird der gewünschte Kronenether in ca. 97 %-iger Reinheit erhalten. Die nach dem erfindungsgemäßen Verfahren isolierten Kronenether enthalten zumeist noch geringe Mengen an Ethylenglycol, das, falls erwünscht, beispielsweise durch azeotrope Destillation mit Toluol entfernt werden kann.

Das erfindungsgemäße Verfahren kann bei allen Synthesen angewandt werden, bei denen Kronenether und Polyglycole im Reaktionsgemisch anfallen. Es sind dies insbesondere Kronenether-Synthesen, die nach Art der Williamson'schen Ethersynthese durchgeführt werden, wobei als Ausgangssubstanzen Polyglycole sowie entsprechende Struktureinheiten aufweisende Halogenide, Tosylate und dergleichen eingesetzt werden.

Das erfindungsgemäße Verfahren ist weiterhin geeignet zur Abtrennung solcher Verbindungen, die sich in Polyglycole umwandeln lassen wie z. B. entsprechende Struktureinheiten aufweisende Vinylether, die oftmals als Nebenprodukte bei der Kronenethersynthese anfallen.

Die Erfindung wird nun anhand von Beipielen näher erläutert:

### Beispiel 1

200 kg eines Reaktionsgemisches, das 58 Gewichtsprozent 18-Krone-6 enthielt und bei der Synthese des genannten Kronenethers aus Tetraethylenglycol, 2,2'-Dichlordiethylether in Gegenwart von KOH und Dimethylsulfoxid als Lösungsmittel anfiel wurden einer Pulsations-Füllkörperkolonne (Ø 80 mm, Länge 6 m) aufgegeben und bei 80°C im Gegenstrom nit ca. 1000 1 Cyclohexan extrahiert. Das Gemisch , das noch 42 Gewichtsprozent an Polyglycolen (Triethylenglycol, Tetraethylenglycol, Pentaethylenglycol, Hexaethylenglycol u.a. polyglycolartige Verbindungen) enthielt wurde im oberen Drittel der Kolonne aufgegeben. Am Kopf der Kolonne wurden 200 kg reines Ethylenglycol zugeführt. Der Extrakt wurde gesammelt, Cyclohexan abdestilliert und 18-Krone-6 in 98 %-iger Reinheit nit einer Ausbeute von 97 % isoliert.

In einem weiteren Reinigungsschritt wurde Toloul zugesetzt und durch azeotrope Destillation nitextrahiertes Ethylenglycol entfernt. Es wurde 18-Krone-6 mit einer Reinheit von 98,6 % (bestimmt mittels Gas-Chronatographie), erhalten.

### Beispiel 2

148 g einer Mischung aus 89 g 18-Krone-6 und 59 g Polyglycolen das bei einer Kronenethersynthese analog Beispiel 1 anfiel, wurdenmit 152 g Ethylenglycol und 900 g Cyclohexan versetzt und bei 80°C 30 Minuten kräftig gerührt. Damach wurde das Gemisch bei 80°C bis zur Phasentrennung stehengelassen. Die spezifisch leichtere Phase wurde abgetrennt und schließlich Cyclohexan abgezogen. Es wurde 18-Krone-6 in 97 %-iger Reinheit erhalten.

### Vergleichsbeispiel 1

Es wurde die Arbeitsweise gemäß Beispiel 2 wiederholt, mit der Abänderung, daß kein Ethylenglycol zugesetzt wurde.

Die Reinheit von 18-Krone-6 betrug 86 %.

## Patentansprüche

1. Verfahren zur Isolierung von makrozyklischen Polyethern aus deren Gemischen mit offenkettigen Polyglycolen durch Extraktion mit gesättigten Kohlenwasserstoffen, dadurch gekennzeichnet daß die Extraktion in Gegenwart von mindestens 10 Gewichtsprozent Ethylenglycol, bezogen auf das Gewicht des Gemisches an makrozyklischen Polyethern/offenkettigen Polyglycolen, durchgeführt wird.

2. Verfahren nach Anspruch 2, dadurch gekennzeichnet, daß die Menge an eingesetztem Ethylenglycol, bezogen auf die Menge des Gemisches an makrozyklischen Polyethern/offenkettigen Polyglycolen 50 bis 200 Gewichtsprozent beträgt.

## Claims

1. Process for isolating macrocyclic polyethers from their mixtures with open-chain polyglycols through extraction with saturated hydrocarbons, characterized in that the extraction is carried out in the presence of at least 10 per cent by weight of ethylene glycol, relative to the weight of the mixture of macrocyclic polyethers/ open-chain polyglycols.

2. Process according to claim 2, characterized in that the amount of ethylene glycol employed is 50 to 200 per cent by weight, relative to the amount of the mixture of macrocyclic polyethers/open-chain polyglycols.

## Revendications

1. Procédé pour isoler des polyéthers macrocycliques à partir de mélanges qui contiennent de tels composés et des polyglycols à chaîne ouverte, par extraction au moyen d'hydrocarbures saturés, procédé caractérisé en ce qu' on effectue l'extraction en présence d'au moins 10 % en poids d'éthylène-glycol, par rapport au poids du mélange de polyéthers macrocycliques et de polyglycols à chaîne ouverte.

2. Procédé selon la revendication 1 caractérisé en ce que la quantité de l'éthylène-glycol utilisé, par rapport à la quantité du mélange de polyéthers macrocycliques et de polyglycols à chaîne ouverte, est comprise entre 50 et 200 % en poids.